# EUROPEAN PATENT SPECIFICATION

(11) **EP 1 194 537 B1**
(45) Date of publication and mention of the grant of the patent: **25.10.2006**
(21) Application number: 00946672.3
(22) Date of filing: 29.06.2000
(51) Int. Cl.: C12N 15/00

(54) **MUTATED NURR1 GENE**
MUTIERTES NURR1 GEN
GENE NURR1 MUTE

(30) Priority: 30.06.1999 SE 9902489
(43) Date of publication of application: 10.04.2002
(73) Proprietor: AstraZeneca AB, 151 85 Södertälje (SE)
(72) Inventor: BUERVENICH, Silvia, S-182 53 Danderyd (SE); OLSON, Lars, S-181 43 Lidingö (SE); ANVRET, Maria, S-175 47 Järfälla (SE); CARMINE, Andrea, S-168 32 Bromma (SE)
(86) International application number: PCT/SE2000/001380
(87) International publication number: WO 2001/000807

(56) References cited:
- US-A- 5 783 680
- ROLF H. ZETTERSTROM ET AL.: 'Dopamine neuron agenesis in nurr1-deficient mice' SCIENCE vol. 276, 11 April 1997, pages 248 - 250, XP002933673
- ODILA SAUCEDO-CARDENAS ET AL.: 'Nurr1 essential for the induction of the dopaminergic phenotype and the survival of ventral mesencephalic late dopaminergic percursor neurons' PROC. NATL. ACAD. SCI. USA vol. 95, March 1998, pages 4013 - 4018, XP002933674

## Description

### Technical field

The present invention relates to novel mutations identified for the first time in the Nurr1 gene.

### Background

Clinical conditions associated with defects of specific genes may be inherited or have alternatively been caused by a later developed mutation in the afflicted individual. It is assumed that many neurological and psychiatric conditions have hereditary components, and indeed, schizophrenia, bipolar affective disorder and Parkinson's disease have in common that affected relatives constitute the largest epidemiological risk factor. However, the mode of inheritance of susceptibility is complex and has not yet been elucidated. A further common observation in said three different diseases is etiologic and/or therapeutic involvement of dopaminergic neuro-transmission. Thus, cells of the mesostrial midbrain dopamine (DA) neuron system degenerate in Parkinson's disease (Hornykiewicz, O. Biochemical aspects of Parkinson's disease. *Neurology **51,*** S2-S9 (1998)), while the mesolimbic DA axis is the target of several antipsychotic drugs (Gerner, R.H., Post, R.M. & Bunney, W.E.J. A dopaminergic mechanism in mania, *Am J. Psychiatry* **133,** 1177-1180 (1976); and Creese, I., Burt, D.R. & Snyder, S.H. Dopamine receptor binding predicts clinical and pharmacological potencies of antischizophrenic drugs. *Science* **192**, 481-483 (1976)). Recently, a number of studies have pointed out the importance of retinoids and retinoid-related genes for DA cells. Midbrain DA neurons express receptors for retinoic acid-mediated transcription, and mice lacking the retinoid-related receptor Nurr1 fail to develop mesencephalic DA neurons (Zetterström, R.H. *et al.* Dopamine neuron aenesis in Nurr1-deficient mice. *Science* **276,** 248-250 (1997).

The immediate early gene Nurr1 (also called *NOT* in humans, classified as NR4A2 according to the most recent nomenclature [Nuclear Receptors Nomenclature Committee, 1999]) codes for a nuclear orphan receptor of the NGFI-B family of transcription factors (Gerner et al., *supra*; Hazel, T.G., Nathans, D. & Lau, L.F. A gene inducible by serum growth factors encodes a member of the steroid and thyroid hormone receptor superfamily. *Proc Natl Acad. Sci.* USA **85**, 8444-8448 (1988); and Mages, H.W., Rilke, O., Bravo, R., Senger, G, G. & Kroczek, R.A. NOT, a human immediate-early response gene closely related to the steroid/thyroid hormone receptor NAK1/TR3. *Mol. Endocrinol.* **8**, 1583-1591 (1994)). The major splice variant is a 598 amino acid protein with ligand binding, DNA binding and N-terminal domains. Recently, further alternatively spliced mRNA variants encoding the entire N-terminal and DNA binding domains but shorter C-terminal domains have been isolated (Ichinose, H. Et al. Molecular cloning of the human Nurr1 gene: characterization of the human gene and cDNAs. *Gene* **230**, 233-239 (1999); Torii, T., Kawarai, T., Nakamura, S. & Kawakami, H. Organization of the human orphan nuclear receptor Nurr1 gene. *Gene* **230,** 225-232 (1999); and Ohkura, N., Hosono, T., Maruyama, K., Tsukada, T. & Yamaguchi, K. An isoform of Nurr1 functions as a negative inhibitor of the NGFI-B family signaling. *Biochim. Biophys. Acta* **1444,** 69-79 (1999)). While no activating ligand for Nurr1 has been identified to date, it has been shown that Nurr1 can be both constitutively active as a transcription factor and dimerize with RXR, a receptor involved in mediating retinoic-acid induced transcription (Perlmann, T. & Jansson, L. A novel pathway for vitamin A signaling mediated by RXR heterodimerization with NGFI-B and Nurr1. *Genes Dev.* **9**, 769-782 (1995)). These studies together with other observations have made Nurr1 a suggestive candidate gene for diseases with an involvement of the dopamine neuron system, such as schizophrenia, manic depressive illness and Parkinson's disease. However, up to date, no evidence has been provided showing any such involvement, and thus, no effective cures have yet been developed based on such genetic knowledge. As about 1% of the GDP of an industrial country each year is spent on patients suffering from diseases such as schizophrenia and bipolar affective disorder, a clear understanding of the genetic background could hopefully lead to effective treatment schemes and diagnoses. Accordingly, within this field, there is a strong need based both on human and economical factors of gaining a more thorough understanding.

Previously, certain genes associated with specific, mental conditions have been disclosed. For example, US 5 783 680 discloses monoxidase genes and proteins associated with abnormal behavior. More specifically, the conditions defined therein are behavioural disorders, such as impulsive aggression, and include borderline mental retardation and aggressive outbursts, often in response to anger, fear or frustration. Such aggressive behaviour was noted to vary markedly in severity. Other types of impulsive behaviour that occurred in individual cases included arson, attemted rape, and exhibitionism. Thus, as is obvious to one of skill in this field, there are clear differences between the conditions which US 5 783 680 relates to and phsyciatric disease, such as schizophrenia. This is evident from the fact, that none of the symptoms cited in said US 5 783 680 are found in the DSM-IV diagnostic criteria for schizophrenia or manic depression, nor are the minimal features required for schizophrenia or manic depression present in the family mentioned in US 5 783 680. (For the DSM-IV criteria, see Diagnostic and Statistical Manual of Mental Disorders, Fourth Edition. Copyright 1994 American Psychiatric Association.) Thus, US 5 783 680 does not relate to mutations that can cause psyciatric disturbances.

Finally, Zetterström et al (Science, vol. 276, 11 April, 1997: "Dopamine Neuron Agenesis in Nurr1-Deficient Mice") have shown that mice that lack Nurr1 fail to generate midbrain dopaminergic neurons and suggest Nurr1 ligands as useful for the treatment of Parkinson's disease.

### Summary of the invention

For the first time, the present invention provides three mutations of the human Nurr1 gene as defined in the appended claim 1 and further shows the relation thereof with a previously unreported subgroup of ca. 1% of the conditions schizophrenia and bipolar affective disorder. The novel mutations according to the invention are herein denoted M1, M2 and M3.

### Brief description of the drawings

Figure 1 is an overview of the genomic structure of the human Nurr1 gene, distribution of PCR fragments 0-7 and localization of mutations M1-M3 according to the invention in exon 3.
Figure 2 shows autoradiographs of the three heterozygous mutations M1-M3 according to the invention.
Figure 3 shows in table 1 the primers used to amplify the PCR fragments 0 to 7.
Figure 4 provides by table 2 clinical descriptions of the three heterozygous carriers of mutations M1 to M3 according to the invention.

### Definitions

In the present application, the term Nurr1 is intended to encompass both mammalian Nurr1 genes, present for example in humans or rodents, and Nurr1 genes of non-mammalian species, such as zebrafish or lamprey. Further, all genes, gene fragments or cDNA species identical to Nurr1 and its cDNA, but published under other names (for example NOT (in humans), RNR1 (in rats) and HZF-3 (in rats)) are also encompassed by the terma Nurr1 as used herein. Thus, any mode of writing such as Nurr1, *Nurr1,* NURR1 and *NURR1* as used herein is to be understood to encompass all of these aspects.

In the present application, the term "nucleic acid" refers to a deoxyribonucleotide or ribonucleotide polymer in either single- or double-stranded form, and encompasses known analogs of natural nucleotides that can function in a similar manner as naturally occurring nucleotides. When reference is made to the gene, it is to be understood that both DNA and cDNA are encompassed.

The term "variant" as used herein means any functional equivalents of the Nurr1 gene including naturally occurring genes present in other species, and mutants, whether naturally occurring or engineered.

In the present specification, the term "highly stringent conditions" means hybridisation to filter-bound DNA in 0.5M NaHPO₄, 7% sodium dodecyl sulfate (SDS), 1 mM EDTA at 65°C and washing in 0.1x SSC/0.1% SDS at 68°C (Ausubel F. M. *et al.,* eds., 1989, *Current Protocols in Molecular Biology,* Vol. I, Green Publishing Associates, Inc., and John Wiley & sons, Inc., New York, at p. 2.10.3). In instances wherein the nucleic acid molecules are deoxyoligonucleotides ("oligos"), highly stringent conditions may refer, e.g., to washing in 6xSSC/0.05% sodium pyrophosphate at 37°C (for 14-base oligos), 48°C (for 17-base oligos), 55°C (for 20-base oligos), and 60°C (for 23-base oligos). Low stringency conditions are well known to those of skill in the art, and will vary predictably depending on the specific organisms from which the library and the labeled sequences are derived. For guidance regarding such conditions see, for example, Sambrook *et al.,* 1989, *Molecular Cloning, A Laboratory Manual,* Cold Springs Harbor Press, N.Y.; and Ausubel *et al.,* 1989, *Current Protocols in Molecular Biology,* Green Publishing Associates and Wiley, Interscience, N. Y.

### Detailed description of the invention

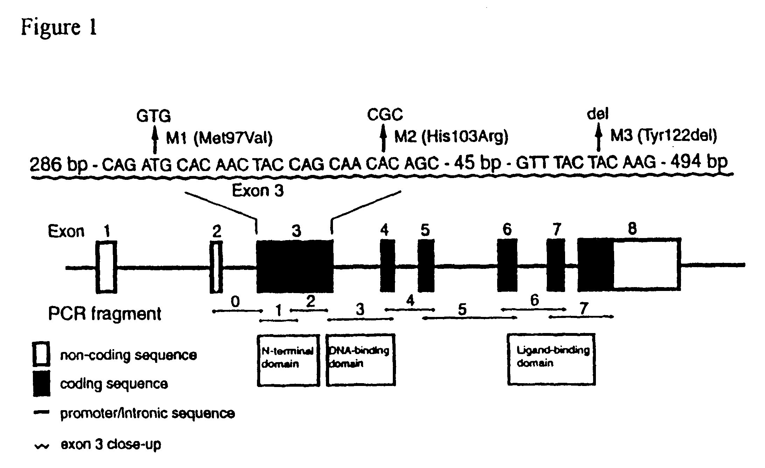

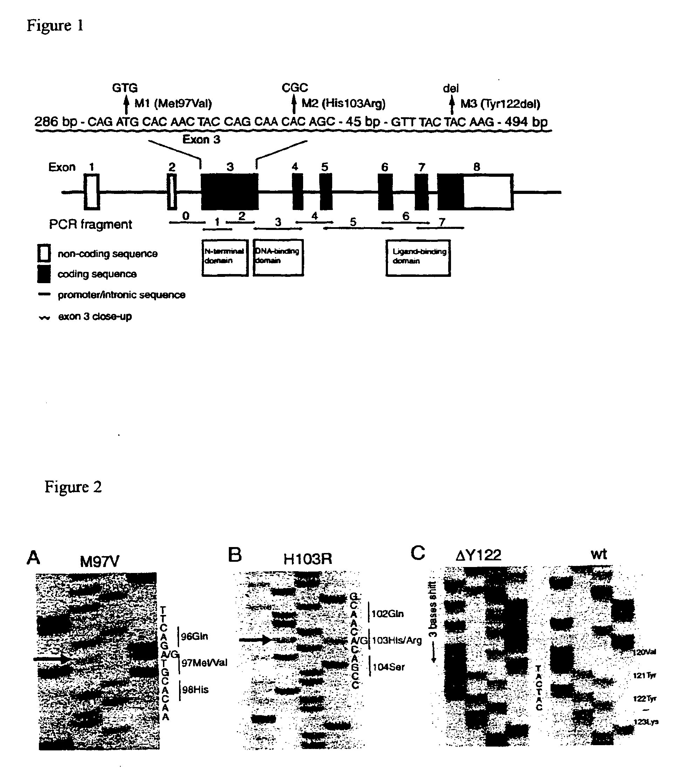

Thus, in a first aspect, the present invention relates to an isolated Nurr1 gene including one or more mutations selected from the group consisting of Met97Val M97V), His103Arg (H103R), Tyr121del (Y121del) and Tyr122 (Y122del). In this context, reference is made to Figure 1 showing an overview of the genomic structure of Nurr1 and the mutations according to the invention. The human Nurr1 gene is comprised of 8 kb as 8 exons and 7 introns located on chromosome 2q22-23, see Ichinose et al., *Gene* **230** (1999) 133-239, which gene has been deposited in the DDBJ/EMBL/GenBank with accession number AB017586. (For the entire sequence of mouse Nurr1, see Castillo *et al., Genomics* **41,** 250-257 (1997), Fig 1, accession no. U86783.) In one embodiment, a fragment according to the invention comprises all the exons of the Nurr1 gene, *i.e* all of the cDNA sequence thereof. More specifically, as the herein defined novel mutations are present on exon 3, in one embodiment, a fragment according to the invention comprises at least exon 3 of Nurr1. Specific embodiments of the first aspect are fragments comprising the mutation Met97Val, wherein a G in the first position of codon no 97 has substituted an A in said position, the mutation His103Arg, wherein a G in the first position of codon no 103 has substituted an A in said position, or the mutation Tyr121del or Tyr122del, wherein a tyrosine has been deleted at position 121 or 122. As easily appreciated by the skilled in this field, and as further discussed in relation to figure 2, the last mentioned deletions are impossible to differentiate from each other, and therefore they will be denoted Tyr122 herein. The present fragments may include any suitable length of the Nurr1 gene depending on the intended use. Further, in the present context, it is noted that the present invention relates to mutated Nurr1 genes of human and mouse origin, irrespective of whether it is written in capital letters or not. Various advantageous uses of the present genes and fragments will be discussed in further detail below.

Accordingly, by disclosing the novel mutations defined above, the present invention shows for the first time a linkage between the Nurr1 gene and neuro-psychiatric disorders, which linkage has previously been sought for without any success, see e.g. Ichinose *et al., supra.* Previously, when such a possible link was discussed, a general connection to neuro-psychiatric conditions was suggested but never substantiated. However, it has never been proposed or foreseen that such a link would exist only to a specific selection of said conditions. Accordingly, the evidence presented herein that mutations in the Nurr1 gene are linked to schizophrenia and manic depressive illness, but not to Parkinson's disease, is highly surprising and will enable an essential step forward in studies aimed at developing new drugs as well as new diagnostic tools within this field. Furthermore, the invention is of importance for the diagnostic field of schizophrenia and bipolar affective disorder because the finding of Nurr1 mutations in a subgroup of patients will help to better characterize the ill-defined and inhomogenous group of patients suffering from these conditions. Thus, those patients suffering from schizophrenia or bipolar affective disorder because of mutated Nurr1 can now be identified and treated differently and more specifically according to their specific genetic defect, which has been revealed for the first time in the present invention.

Thus, even though Zetterström et al (Science, 1997, supra) suggested that putative Nurr1 ligands could be useful for the treatment of Parkinson's disease and other disorders of midbrain dopamine circuitry, they could not show any link between Nurr1 and the herein discussed conditions of schizophrenia and manic depression. The present invention discloses for the first time three specific mutations which as shown below in the experimental section are clearly linked to a subgroup of patients suffering from said conditions. Although the three disclosed mutations cannot explain the condition in all schizophrenia or manic depressed patients, their absence from investigated control material strengthens their role in the patients where they have been identified. Since an effect of each one of the mutations on Nurr1 activity has been shown, and all three mutations show very similar effects, reliable evidence is provided that the present mutations contribute to the phenotype observed in those patients in which mutations were found.

To further stress the practical usefulness of the present invention, it is noted that a person skilled in the art is well aware of the multifactorial etiology of complex diseases, such as schizophrenia and manic depression. It is therefore unlikely that one would find mutations in a single gene in a large portion of patients. Given a worldwide prevalence of both diseases of about 1% each, explaining 1% of these diseases can help an enormous number of people.

Actually, after above discussed description of mutations in MAOA in patients with impulsive aggression (US 5 783 680), another study (Am J Med Genet 1999 Feb 5;88(1):25-8)was carried out searching for MAOA mutations in patients with similar diagnoses (and also healthy individuals and Parkinson patients who served as control material), without finding any muations. Thus, similarly to the novel mutations according to the present invention, MAOA mutations are very rare causes of certain conditions, which in their case are behavioural.

The present invention further discloses a nucleic acid capable of specifically hybridising to a gene or a fragment as defined above. The preparation of DNA is easily accomplished by the skilled in this field by any suitable method, such as cloning and restriction of appropriate sequences by any *in vitro* or *in vivo* method, such as the polymerase chain reaction (PCR), the ligase chain reaction (LCR), the transcription-based amplification system (TAS), the self-sustained sequence replication system (SSR) or cell based cloning and amplification, wherein the cell may be any suitable cell, such as a bacterium, a cultured cell line etc. A wide variety of cloning and amplification methods are well known to the skilled in this field, see e.g. Sambrook *et al.,* (1989); *Molecular Cloning: A Laboratory Manual,* 2^{nd} Ed, vol. 1-3). Further, they may be prepared by direct chemical synthesis by methods such as the phosphotriester method (Narang *et al., Meth. Enzymol.* **68,** 90-99 (1979); the phosphodiester method (Brown *et al., Meth. Enzymol.* **68,** 109-151 (1979); the diethylphosphoramidite method (Beaucage *et al., Tetra. Lett.,* **22,** 1859-1862 (1981); and the solid support method (US patent no. 4 458 066).

The present fragments may e.g. be used as primers, e.g. for PCR, which for example may be present in a kit, as probes etc. Further, as discussed below, they may be used to generate immunogenic polypeptides or fusion proteins for use in generating specific antibodies which recognise the mutant epitope. For use as primers, the fragments are preferably of a length of at least about 10, such as at least about 15-20, bases, which however depends on the method used.

The present invention further discloses a vector comprising a nucleic acid according to the invention as well as to cells harbouring such a nucleic acid useful for the production of the present protein or peptide. Such cells may be procaryotic or eucaryotic and are described in more detail below in relation to cell culture model systems.

Another aspect of the invention is a protein or a peptide encoded by a gene or a gene fragment according to the invention. Accordingly, in specific embodiments, the present protein or polypeptide may include a Val residue in the position corresponding to amino acid no. 97 of the wild type Nurr1 protein; an Arg residue in the position corresponding to amino acid no. 103 of the wild type Nurr1 protein; and/or a sequence that does not include any Tyr residue in the position corresponding to amino acid no. 122 of the wild type Nurr1 protein.

Further, the present protein or polypeptide may also be used to specifically design a substance binding thereto and capable of inhibition or other alteration of the function thereof.

The present invention also relates to methods of treatment as well as to the use of the present proteins or peptides in the manufacture of a medicament. Further, the present proteins or peptides are also useful in diagnosis of schizophrenia and/or manic depressive disease, as the expression thereof in a patient will be an indication of the existance of one of the mutations according to the invention.

A further aspect of the invention is an antibody which specifically binds to a protein or peptide according to the invention and preferably comprises at least about 10, more preferably at least 20, 40 or 50 or more amino acids. Such an antibody may be used as a medicament in order to inhibit or suppress the biological effect of the product of a mutated gene. Alternatively, the present antibody may be used in the diagnosis of schizophrenia and/or manic depressive disorder. The present antibody may be polyclonal or monoclonal, preferably, it is a monoclonal antibody. It can be humanized or human The invention also relates to a cell, such as a recombinant cell, e.g. hybridomas or triomas, expressing such an antibody as defined above. Methods of producing polyclonal antibodies are known to those of skill in the art In brief, an immunogen (antigen), preferably a purified polypeptide, a polypeptide coupled to an appropriate carrier (*e.g.,* GST, keyhole limpet hemanocyanin, *etc.*), or a polypeptide incorporated into an immunization vector such as a recombinant vaccinia virus (*see,* U.S. Patent No. 4,722,848) is mixed with an adjuvant and animals are immunized with the mixture. The animal's immune response to the immunogen preparation is monitored by taking test bleeds and determining the titer of reactivity to the polypeptide of interest When appropriately high titers of antibody to the immunogen are obtained, blood is collected from the animal and antisera are prepared. Further fractionation of the antisera to enrich for antibodies reactive to the polypeptide is performed where desired (*see, e.g.,* Coligan (1991) *Current Protocols in Immunology* Wiley/Greene, NY; and Harlow and Lane (1989) *Antibodies: A Laboratory Manual* Cold Spring Harbor Press, NY).

Monoclonal antibodies are prepared from cells secreting the desired antibody. These antibodies are screened for binding to normal or modified polypeptides, or screened for agonistic or antagonistic activity. Specific monoclonal and polyclonal antibodies will usually bind with a K_{D} of at least about 0.1 mM, more usually at least about 50 µM, and most preferably at least about 1 µM or better. In some instances, it is desirable to prepare monoclonal antibodies from various mammalian hosts, such as mice, rodents, primates, humans, *etc.* Description of techniques for preparing such monoclonal antibodies are found in, *e.g.* Stites *et al.* (eds) *Basic and Clinical Immunology* (4th ed.) Lange Medical Publications, Los Altos, CA, and references cited therin; Harlow and Lane, *supra*; Goding (1986) *Monoclonal Antibodies: Principles and Practice* (2d ed.) Academic Press, New York, NY; and Kohler and Milstein (1975) *Nature* 256: 495-497.

The pharmaceutical preparations comprising a substance identified by use of the herein described methods according to the invention, or an antibody as disclosed above, according to the invention may be prepared by any standard method according to standard methods, see e.g. *Remington's Pharmaceutical Sciences,* 16^{th} ed. Osol, A (ed.) 1980; and Langer, *Science* **249**, 1527-1533 (1990).

The peptides, polypeptides and proteins according to the invention may be synthesized using standard chemical peptide synthesis techniques well known to the skilled person. For solid phase synthesis, see e.g. Barany and Merrifield, *Solid-Phase Peptide Synthesis,* in *The Peptides: Analysis, Synthesis, Biology,* vol 2: *Special Methods in Peptide Synthesis.*

The present peptides and polypeptides may also be produced using recombinant DNA technology. Generally, this involves creating a DNA sequence that encodes the desired peptide, i.e., including one or more of the present mutations, e.g. as a recombinant sequence encoding the present peptide as part of a fusion protein, placing the DNA in an expression cassette under the control of a particular promoter, expressing the peptide in a host and isolating the expressed peptide. In case of a protein, the renaturation thereof may be required. The nucleic acids encoding the peptides or proteins may be expressed in a variety of host cells, including E.coli, other bacterial hosts, yeast, and various higher eucaryotic cells, such as COS, CHO and HeLa cell lines and myeloma cell lines. The gene will be operably linked to appropriate expression control sequences for each host. For E.coli, this includes a promoter such as the T7, trp or lambda promoters, a ribosome binding site and preferably a transcription termination signal. For eucaryotic cells, the control sequences will include a promoter and preferably an enhancer and it may include splice donor and acceptor sequences. The expression systems that may be used for the preparation of the present proteins and peptides include but are not limited to microorganisms such as bacteria (e.g., E. coli, B. subtilis) transformed with recombinant bacteriophage DNA, plasmid DNA or cosmid DNA expression vectors containing the present nucleotide sequences; yeast (e.g., Saccharomyces, Pichia) transformed with recombinant yeast expression vectors containing the the present nucleotide sequences; insect cell systems infected with recombinant virus expression vectors (e.g., baculovirus) containing the the present sequences; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing the present nucleotide sequences; or mammalian cell systems (e.g., COS, CHO, BHK, 293, 3T3) harboring recombinant expression constructs containing promoters derived from the genome of mammalian cells (e.g., metallothionein promoter) or from mammalian viruses (e.g., the adenovirus late promoter; the vaccinia virus 7.5K promoter).

Once expressed, standard procedures may be used for purification, such as ammonium sulphate precipitation, affinity columns, column chromatography, gel electrophoresis and the like. See, generally, R. Scopes, *Protein Purification,* Springer-Verlag, N.Y. (1982), Deutscher, *Methods in Enzymology,* Vol 182: *Guide to protein purification,* Academic Press, Inc., N.Y. (1990).

The present invention further discloses a cell culture comprised of transgenic cells harbouring one or more of the present mutations for use as a model, e.g. for testing novel pharmaceutically active substances. Such cells may carry one or more of the present mutations on a vector or in the genome. In one embodiment, they are isolated cells that have been mutated spontaneously in a natural environment, while another embodiment relates to cells produced by recombinant DNA techniques. Such a cell culture will comprise immortalized cells, such as primary nerve cells, COS or HeLa cells etc. Any one of the various methods currently used for the introduction into and amplification of foreign (heterologous) nucleic acid into cultured cells for research purposes as well as for the purpose of overproduction of proteins of interest for pharmaceutical applications may be used according to the invention. The present cell lines comprise inserted genes or gene fragments as described above. Alternatively, they are derived from a transgenic animal (see e.g. Babinet *et al.,* US patent no. 5 073 490). Transgenic animals are disclosed in further detail below, but a general review of the basic process for the creation of transgenic animal particularly mice, is found e.g. in Palmiter *et al.,* in "Dramatic Growth of Mice That Develop From Eggs Microinjected with Metallothionein-Growth Hormone Fusion Genes", *Nature.* Vol. **300,** 611-615 (Dec. 1982).

However, mammalian systems are the most preferred. In mammalian host cells, a number of viral-based expression systems may be utilized. In cases where an adenovirus is used as an expression vector, the nucleotide sequence of interest may be ligated to an adenovirus transcription/translation control complex, e.g., the late promoter and tripartite leader sequence. This chimeric gene may then be inserted in the adenovirus genome by *in vitro* or *in vivo* recombination. Insertion in a non-essential region of the viral genome will result in a recombinant virus that is viable and capable of expressing the present gene product in infected hosts. (See e.g. Logan & Shenk, 1984, *Proc. Natl. Acad. Sci.* USA **81:**3655-3659). Specific initiation signals may also be required for efficient translation of the present inserted nucleotide sequences. These signals include the ATG initiation codon and adjacent sequences. In cases where an entire Nurr1 gene or cDNA, including its own initiation codon and adjacent sequences, is inserted into the appropriate expression vector, no additional translational control signals may be needed. However, in cases where only a portion of the coding sequence is inserted, exogenous translational control signals, including, perhaps, the ATG initiation codon, must be provided. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (See Bittner *et al.,* 1987, *Methods in Enzymol.* **153,** 516-544). Further, a purification of the present product is needed. For example, an antibody specific for the expressed peptide or protein may be used. For example, a system described by Janknecht *et al.* allows for the ready purification of non-denatured fusion proteins expressed in human cell lines (Janknecht, *et al.,* 1991, *Proc. Natl. Acad. Sci.* USA **88**, 8972-8976).

Further, the present invention relates to a transgenic animal, preferably a non-human animal, such as a mammalian, e.g a mouse, comprising a mutated gene or a gene fragment according to the invention, as well as to any further generation of said animal. For a comprehensive instruction on how animal models can be established for human diseases with identified genetic mutations, reference is made to Human Molecular Genetics, BIOS Scientific Publishers Limited, 1996, chapter 19, pp. 507-550, entitled "Studying human gene structure and function and creating animal models of disease", Strachan, T. and Read, A.P. Any technique known in the art may be used to introduce the present gene comprising one or more of the novel mutations into animals to produce the founder lines of transgenic animals. Such techniques include, but are not limited to pronuclear microinjection (Hoppe, P.C. and Wagner, T. E., 1989, U.S. Pat. No. 4,873,191); retrovirus mediated gene transfer into germ lines (Van der Putten *et al.,* 1985, *Proc. Natl. Acad. Sci.,* USA **82**:6148-6152); gene targeting in embryonic stem cells (Thompson *et al.,* 1989, *Cell* **56**:313-321); electroporation of embryos (Lo, 1983, *Mol Cell. Biol.* **3**:1803-1814); and sperm-mediated gene transfer (Lavitrano *et al.,* 1989, *Cell* **57**:717-723); etc. For a review of such techniques, see Gordon, 1989, Transgenic Animals, *Intl. Rev. Cytol.* **115**:171-229. Most preferably, the transgenic animal according to the invention is a mouse comprising one or more of the present mutations M1-M3.

Alternatively, instead of introducing a complete gene or gene fragment into the host, the desired mutation(s) is provoked in a laboratory animal, such as a mouse. This may be accomplished by any mutagenic process, including in vitro-mutagenesis followed by transfer of genomic material into embryonic stem cells or any other mutagenic process, including chemical, biological, radioactive, or ultraviolet treatments.

Due to the fact that the domains of the Nurr1 gene surrounding M1, M2 and M3 are highly conserved in mouse and human, see e.g. Ichinose *et al., supra,* the most preferred transgenic animal model according to the invention is a mouse, wherein one or more of the present mutations M1-M3 have been introduced. Thus, for reasons of simplicity in the preparation, the mouse model is an ideal candidate for studies of the present human mutations.

Methods of transferring genes into the germline, the expression of natural and hybrid genes and phenotypic changes that have occurred in transgenic mice are described by Palmiter and Brinster in *Ann. Rev. Genet.* **20** (1986) 465-499.

Accordingly, the present invention also relates to the use of an animal model as defined above for the identification of new drugs. More specifically, such a method of testing the therapeutic activity of a pharmacological agent on schizophrenia or manic depressive illness comprises administering an effective amount of said pharmacological agent to the mouse model according to the invention and evaluating said agent's effect on said mouse. Any substance identified by use of a model according to the invention is also within the scope of the invention.

Another aspect of the invention is a method of detecting the presence of a mutation in exon 3 of the Nurr1 gene, which mutation is selected from the group consisting of Met97Val, His103Arg, Tyr121del and Tyr122del, which method comprises obtaining a biological sample from e.g. a human subject or an animal model and analyzing said sample for said mutation. More specifically, the biological sample is analyzed by isolating DNA from said sample, amplifying said DNA, hybridising said DNA to a labeled oligonucleotide probe that specifically hybridizes to mutant DNA containing a G as the first base of codon no 97; a G as the second base of codon no 103; or (how do I say this:) altered TACTAC in codon no 121 or 122, or to one or more bases adjacent to said mutation, depending on the method used. Methods of detecting mutations in DNA are e.g. reviewed in U. Landgren, GATA 9, 1992, pp. 3-8.

Further, the present mutations in the Nurr1 gene can be detected by either of the following methods (a)-(j), which are described in detail in: (1) *Mutation Detection, A Practical Approach,* R.G.H. Cotton, E. Edkins and S. Forrest (editors), The Practical Approach Series, Oxford University press (1998) and (2) *Finding Mutations, The Basics,* J.R. Hawkins, Oxford University Press (1997).

(a) Single strand conformation polymorphism analysis (SSCA, also called SSCP), alone or in combination with heteroduplex analysis (HA) is one of the most widely used approaches for mutation detection. (b) Denaturing gradient gel electrophoresis (DGGE) is the name of a whole family of similar methods that are based on the reduction in electrophoretic mobility of a DNA molecule in a dense medium during denaturation. (c) The ribonuclease protection assay or RNase cleavage assay (RPA), the chemical cleavage of mismatch (CMM) assay and mutation detection using T4 endonuclease VII (EMC) are effective methods to detect DNA-DNA or DNA-RNA mismatches caused by mutations. (d) Hybridisation with sequence specific oligonucleotide probes (SSOP) takes advantage of the fact that under stringent conditions, a single basepair mismatch can prevent hybridisation of short complementary oligonucleotide probes. (e) Ligation assays such as the oligonucleotide ligation assay (OLA) comprise further ways of detecting changes in DNA sequences. (f) Direct sequencing, which constitutes one of the most sensitive methods of mutation detection. (g) A rather new method originating from direct sequencing is called minisequencing or solid phase minisequencing. (h) Selective amplication of specific alleles (PASA, also called ASA or ASP) makes it possible to detect mutations already during amplification of the fragment of interest by PCR. (i) With the protein truncation test (PTT), one can identify mutations that induce stop codons in DNA sequence after translation of the sequence into protein. (j) Restriction fragment length polymorphism (RFLP) analysis makes use of endonucleases which recognize and cleave specific nucleotide sequences. Mutations in DNA can either change the sequence so that an endonuclease which cuts the wildtype sequence cannot recognize and cut the mutated sequence, or it can induce a new cleavage site for an endonulease not cutting the wildtype sequence. In sequences that cannot be distinguished by naturally occurring enzymes, mutations can be analyzed by primer-introduced restriction analysis, a method which alters the sequence surrounding the mutation.

In addition, the invention also relates to a kit for performing any one of the methods described above. Accordingly, essential agents for mutation detection according to the respective method can be combined in single packages as user-friendly kits for easy mutation detection. Such kits may contain reagents and enzymes for amplification of the mutated sites, oligonucleotides hybridizing to DNA sequences around and/or containing the mutations and enzymes for specific cleavage of DNA at mutated sites. Further, the present kits may also include suitable labels. The present invention also relates to the production of any such kit aimed at detecting the mutations M1-M3 identified by the inventors.

Finally, the present invention also relates to methods of gene therapy aimed at correcting one or more of the mutations according to the invention. Techniques for gene therapy are well known to the skilled artisan. For example, WO 93/24640 and WO 95/11984 disclose methods and compositions for *in vivo* gene therapy using nonviral or viral vector technology. In another example, WO 95/06743 discloses a method whereby therapeutic nucleic acid constructs are introduced into a patient's isolated airway epithelial cells via transformation with a viral vector containing a construct. The transformed cells are then administered to the patient. The technology for the various nonviral or viral vectors, that can be applied to cells *in vitro* or injected or applied *in vivo* to patients are likewise well known to the skilled artisan.

### Detailed description of the drawings

Figure 1 is an overview of the genomic structure of the human Nurr1 gene, distribution of PCR fragments 0-7 and localization of the mutations M1-M3 according to the invention in exon 3. All three mutations are localized within 78 basepairs of PCR fragment 1 comprising the first 495 bases of exon 3. Fragment 0 was only sequenced once in order to design a new primer within the second intron closely adjacent to exon 3. All other fragments (1-7) were used for the sequencing of the coding region in the first 20 Parkinson patients, 20 schizophrenics and 4 controls. After identification of the first mutation, only fragment 1 was sequenced in all further patients.
Figure 2 shows autoradiographs of the three heterozygous mutations M1-M3 according to the invention. *a,* M1 (M97V) leads to a double band at the first nucleotide of the codon for amino acid position 97. The arrow points at the wildtype band. *b,* M2 (H103R) exchanges the middle nucleotide in the codon for amino acid 103. The arrow indicates the wildtype. *c,* M3 (ΔY122) deletes one of the tyrosines at positions 121 or 122. It is not possible to tell which three consecutive bands have been deleted. However, the pattern of the 3 basepair shift beginning at the TACTAC sequence must result from a 3 basepair deletion within these six basepairs and the surrounding amino acid sequence is not affected by this shift. Because a reverse primer was used for generating this autoradiograph, the shift occurs in the adjacent sequence above the double tyrosine codons. On the right, the wildtype (wt) sequence is shown for comparison.
Figure 3 shows in table 1 the primers used to amplify the PCR fragments 0 to 7. Because no intronic sequences were known when the present study was initiated, primers are located in exons and almost all fragments contain intron sequence. More specifically, fragment no. 0 comprises a part of the second (non-coding) exon, intron 1 (complete) and 2 bases of exon 3; in fragment no. 1, the forward primer is located in the second intron, immediately adjacent to the border to the third exon, and this fragment contains the first 513 bases of exon 3; fragment no. 2 is the second half of exon 3, both primers being located within coding sequence; fragment no. 3 is the rest of exon 3, complete intron 3 and major part of exon 4; fragment no.4 comprises exon 4, intron 4 and exon 5; fragment no.5 comprises exon 5, intron 5 and exon 6; fragment no.6 comprises exon 6, intron 6 and exon 7; and fragment no.7 comprises exon 7, intron 7 and exon 8 including 34 bases of 3' untranslated region.
Figure 4 provides by table 2 clinical descriptions of the three heterozygous carriers of mutations M1 to M3 according to the invention. In table 2, the following abbreviations are used: MD: Major depression; SAP: Separation Anxiety Disorder; ADHD: Attention Deficit/Hyperactivity Disorder. More specifically, the history of the female heterozygous carrier of M1 is an onset with auditary hallucinations, later displayed delusions of reference, paranoid delusions, verbal auditory hallucinosis, visual and tactile hallucinosis, thought insertion and thought "broadcast", as well as flat and sometimes inappropriate affects. She responded well to antipsychotic treatment but experienced relapses after discontinuation of the antipsychotic medication. She is currently on continuous antipsychotic medication and has been free from psychotic episodes for several years. Her family history was as follows: The paternal grandmother's sister had been treated at a mental hospital, the cause of illness unknow. The history of the M2 carrier includes a first hospital admission after 5 months of expansive and irritable mood, decreased need for sleep, distractability, and excessive involvement in pleasurable activities. The end of this period also included psychotic symptoms: verbal mood-congruent auditory hallucinations, delusions of reference, grandiose delusions, incoherence, and disorganized behaviour. Treatment was performed with antipsychotic medication and discharge to an outpatient department. Rehospitalization took place a month later due to depressed mood, weight gain, loss of energy, feeling of worthlessness, difficulties to concentrate, and recurrent thought of death. No psychotic symptoms occurred during later manic and depressive episodes. She has currently been treated with lithium for more than four years without relapses. The family history of the M2 carrying patient was as follows: The patient reported no major psychiatric disturbances in the family history. However, the paternal grandfather was said to be a confidence trickster. The history of the M3 carrier includes two episodes of extended depressed mood and anhedonia (ages 6-7 and 10-11). Auditory hallucinations were experienced since the second episode. Hallucinations and delusions occur regularly and are independent of mood state. Intermittent history of obsessions and compulsions since age of 6. Frequent episodes of illogical thinking and neologisms and short periods of incoherent speech. The family history of the M3 carrying patient was as follows: The mother reported a few occasional (olfactory, visual) hallucinatory episodes but no further symptoms.

### Experimental

The examples below are given merely to illustrate the present invention and are not intended to limit the scope of the invention as defined by the appended claims. All references given below or elsewhere in the present specification are included herein by reference.

### Example 1: Identification of mutations

### Methods

### General

By direct sequencing of genomic DNA, one missense mutation and one deletion of three basepairs in the third exon of Nurr1 were found in two schizophrenic patients. One missense mutation was found in the same exon in an individual with manic depressive disorder. None of these mutations were present in patients with Parkinson's disease or control DNA material of normal populations.

The entire coding region of the Nurr1 gene was first sequenced in 20 patients with Parkinson's disease, 20 patients with schizophrenia and 4 healthy control individuals. The primers used for PCR amplification of the DNA fragments shown in figure 1 are summarized in table 1. One deletion of three basepairs (ΔY122, fig 1 and 2) was identified in one childhood-onset schizophrenic individual originating from the USA. This mutation (M3) was located in fragment 1 which covers about the first half of the N-terminal domain. This mutation was not found when fragment 1 was sequenced in 100 healthy individuals from the USA, nor were any other mutations found in the same fragment in this material. As mutations are frequently clustered in coding regions of disease-causing genes, the present sequencing efforts were continued by focusing on fragment 1. This fragment was sequenced in 60 Swedish controls, 50 additional Swedish Parkinson patients, 17 additional American schizophrenics, 135 additional Swedish schizophrenics and 29 manic depressed patients from the USA and Sweden. One missense mutation (H103R; M2 in the figures) was identified in a Swedish schizophrenic patient and another missense mutation (M97V; M1 in the figures) was identified in a Swedish patient with manic-depressive illness with psychotic symptoms. All three mutations were absent from all other DNA samples and are therefore unlikely to be polymorphic variants of the human Nurr1 gene. Notably, no mutations were found in the Parkinson DNA samples. Attempts to obtain DNA samples from relatives of the three mutation carriers were only moderately successful. Only in the childhood onset case (M3) was it possible to obtain parental DNA. The mother was a healthy carrier of the mutation, but no further family history could be obtained in this kindred. The medical and family histories of all three patients are summarized in relation to table 2, figure 4. Interestingly, all three mutations cluster in evolutionary conserved amino acids in close vicinity to each other in the N-terminal region of Nurr1, which might indicate a common effect on Nurr1 function. Preliminary results from *in vitro* mutagenesis experiments indicate that transcriptional activity mediated by Nurr1 monomer binding to the Nurr1 binding response element is not severely affected by any of the three mutations (data not shown). More complex assays measuring Nurr1 heterodimerization with RXR, Nurr1 homodimerization and interaction of Nurr1 with other activating or silencing factors are needed to elucidate possible functional consequences of the mutations. Because the Nurr1 gene is evolutionary highly conserved and all amino acids affected by the mutations are identical in mice, generation of transgenic mice carrying the novel mutations according to the invention provides additional opportunities to understand how Nurr1 function is affected. The generation of such mice will help elucidating one pathway for the development of schizophrenia and/or bipolar affective disorder and thus will also help to find further genetic and environmental etiological factors for these diseases.

### Patients

All schizophrenic and bipolar affective disorder patients fullfilled the DSM-III-R (Diagnostic and Statistical Manual of Mental Disorders, Third Revised Edition. Copyright 1987 American Psychiatric Association) or DSM-IV (Diagnostic and Statistical Manual of Mental Disorders, Fourth Edition. Copyright 1994 American Psychiatric Association) diagnostic criteria. Parkinson patients were diagnosed according to the "Brain bank clinical criteria" for idiopathic Parkinson's disease (Daniel, S.E. & Lees, A.J. Parkinson's Disease Society Brain Bank, London: overview and research. *J. Neural Transm. Suppl.* **39,** 165-172 (1993)) except that three cases had more than one affected relative but were still included. DNA samples from bipolar affective disorder patients were purchased from the Coriell Cell Repository (Camden, USA). While all schizophrenic and Parkinson patients involved in this study were unrelated individuals, patients with bipolar affective disorder belonged to the Coriell families number 811 (n=3), 812 (n=2), 823 (n=5), 830 (n=4), 834 (n=1) 835 (n=1), 884 (n=5), 888 (n=1), 893 (n=2) and 1075 (n=2). Three manic depressed patients from Sweden were also included in the material as presumed controls or schizophrenic patients. At the present study they turned out to fulfill the DSM-III-R criteria for bipolar affective disorder.

### DNA sequencing

DNA was extracted from whole blood accoring to standard protocols. The genomic structure of the human Nurr1 gene was deduced from the homologous mouse Nurr1 gene, and primers covering the second exon and the entire coding region were designed (table 1, fig 3). The numbering of the nucleotides and amino acids employed herein follows the initial publication of the human Nurr1 *(NOT)* mRNA sequence (Mages *et al., supra*). The sequence of the second intron was determined by sequencing of fragment 0, and one additional forward primer was designed located in the second intron (see table 1, figure 3, and figure 1). Polymerase chain reaction (PCR) was carried out using Taq DNA polymerase (SIGMA). 35 cycles were run at 94°C for 40 seconds, 56°C for 45 seconds and 72°C for one minute. After PCR, the samples underwent electrophoresis on 1% low-melting agarose gels and were visualized using UV-translumination after ethidiumbromide staining. DNA was extracted from gel slices (PCR preps DNA purification kit, SDS) and DNA fragments were sequenced (Thermo Sequenase radiolabeled terminator cycle sequencing kit, Amersham). The reaction products were run on 6% acrylamid sequencing gels (National Diagnostics) and visualized by autoradiography.

### Results

The present invention shows that two out of 187 patients with schizophrenia and one out of 29 patients with bipolar affective disorder carried unique mutations clustered in the area of the N-termina1 domain of the Nurr1 gene. Even though the frequency of mutated Nurr1 determined according to the invention may appear low (about one percent for schizophrenia and three percent for bipolar disorder), it is perfectly in line with the multifactorial origin of these diseases, with a number of different gene defects remaining to be identified explaining a fraction of the total number of patients each.

### Example 2: Function of identified mutations

Because the rarity of molecular variants of NURR1 does not exclude their role in the pathogenesis of psychiatric disease in those individuals where they were identified, functional assays were set up in order to elucidate if any of the three clustered mutations might affect *NURR1* function. The following description of results from such functional studies is adapted from the manuscript entitled *"NURR1* Mutations in Cases of Schizophrenia and Manic Depressive Disorder" by authors Silvia Buervenich, Andrea Carmine, Mariette Arvidsson, Fengqing Xiang, Zhiping Zhang, Olof Sydow, Erik G. Jönsson, Göran C. Sedvall, Sherry Leonard, Randal G. Ross, Robert Freedman, Kodavali V. Chowdari, Vishwajit L. Nimgaonkar, Thomas Perlmann, Maria Anvret, and Lars Olson. The cited work is currently in press in the American Journal of Medical Genetics; Neuropsychiatry Section.

### In-vitro expression assay

Human *NURR1* cDNA sequence was cloned into the expression vector pCMX [Umesono et al., 1991], and expression vectors for the mutants were generated by site-directed mutagenesis (GeneEditor In Vitro Site-Directed Mutagenesis System, Promega). A double stranded NurRE [Philips et al., 1997] DNA fragment was generated by annealing the primers 5'-AGC TTG TGA TAT TTA CCT CCA AAT GCC AG-3' and 5'-AGC TCT GGC ATT TGG AGG TAA ATA TCA CA-3'. A luciferase reporter plasmid containing three tandem NurRE sites was generated by ligating the annealed fragments upstream of the herpes simplex thymidine kinase promoter fused to the luciferase gene. Human embryonic kidney (HEK)-293 cells were grown in Dulbecco's modified Eagle's medium supplemented with 10% fetal calf serum (FCS). Transfections were performed in 24-well plates by the calcium phosphate method. Briefly, cells were seeded one day prior to transfection. Cells in each well were treated with 100ng of the indicated expression vectors, 100ng of reporter plasmid and 200ng of reference CMX-βgal plasmid containing the β-galactosidase gene and CMX-PL1 as carrier DNA up to 500ng of total DNA. Cells were exposed to calcium phosphate precipitate for 12-16h and washed with PBS and then fresh medium was added. The cells were harvested and lysed after 36h incubation. Extracts were assayed for luciferase and β-galactosidase activity in a microplate luminometer/photometer reader (Lucy-1, Anthos). All luciferase activities were normalized to β-galactosidase activity.

NURR1 transcriptional activity was measured using human embryonic kidney (HEK)-293 cells monitoring NURR1 homodimer binding to NurRE [Philips et al., 1997]. Each experiment was carried out using two independent clones for the wildtype and mutated vectors, respectively, and activity was measured in at least four separate wells for each clone. A significant reduction (30-40%) of transcriptional activity of mutated NURR1 homodimers was found. This reduction was strikingly similar in all three mutated clones and is consistent with the present finding that the three mutations are clustered in a region of NURR1 which is critically important for transcriptional activation. The avarage activity and s.e.m. of mutated NURR1 in percent of wildtype activity in all experiments carried out were ΔY122: 0.645 ± 0.024, n=87; H103R: 0.608 ± 0.036, n=87; and M97V: 0.661 ± 0.046, n=54.

The facts that (1) the three mutations were clustered within 78 basepairs of the N-terminal domain, (2) they all lead to impaired NURR1 transcriptional activity in an *in-vitro* bioassay, and (3) except for the three mutations this region of the *NURR1* gene was totally conserved at the DNA level in our entire material of 620 individuals (1240 alleles) suggest that the *NURR1* gene does constitute an interesting gene for mutation screening in diseases with an involvement of the dopamine system.

### Figure 3

**Table 1**

| Fragment number | Description | Primer sequences |
|---|---|---|
| 0 | Part of the second (noncoding) exon, intron 1 (complete) and 2 bases of exon 3 | F - GGAGATTGGACAGGCTGGAC |
| | | R - TGCGCCTGAACACAAGGCAT |
| 1 | The forward primer is located in the second intron, immediately adjacent to the border to the third exon. This fragment contains the first 513 bases of exon 3 | F - TTATCACCCTGTTTCATTTCC |
| | | R - GAGACTGGCGTTTTCCTCT |
| 2 | Second half of exon 3. Both primers are located within coding sequence | F - TGCCGCACTCCGGGTCGGTTTACTACA |
| | | R - GCCCTCACAGGTGCGCACGCCGTA |
| 3 | Rest of exon 3, complete intron 3 and major part of exon 4 | F - CACGCGTCTCAGCTGCTCGACAC |
| | | R - CTTCTTTGACCATCCCAACAGCCA |
| 4 | Exon 4, intron 4 and exon 5 | F - CGCACAGTGCAAAAAAATGCAA |
| | | R - CCTGGAATAGTCCAGGCTGG |
| 5 | Exon 5, intron 5 and exon 6 | F - TGGTTCGCACAGACAGTTTA |
| | | R - GCTAATCGAAGGACAAACAG |
| 6 | Exon 6, intron 6 and exon 7 | F - TTCCAGGCGAACCCTGACTA |
| | | R - ACCATAGCCAGGGCAGCAAT |
| 7 | Exon 7, intron 7 and exon 8 including 34 bases of 3' untranslated region | F - TCCAACCCAGTGGAGGGTAA |
| | | R - ATTCCAGTTCCTTTGAAGTGC |

### Figure 4

**Table 2**

| Mutation number | Diagnosis | Country of origin | Age of onset | Prominent symptoms | Affected family members |
|---|---|---|---|---|---|
| M1 | Schizophrenia | Sweden | 26 | Auditory hallucinations, delusions of reference, and paranoid delusions. | Paternal grandmother's sister |
| M2 | Bipolar disorder with psychotic features | Sweden | 21 | Auditory hallucinations, delusions of reference, incoherence and depressed mood. | Paternal grandfather |
| M3 | MD (past); Childhood-onset schizophrenia, SAP, ADHD (current) | USA | 6 | Extended depressed mood, anhedonia, auditory hallucinations and delusions. | Not known |

## Claims

1. An isolated Nurr1 gene including one or more mutations selected from the group consisting of Met97Val (M97V), His103Arg (H103R), Tyr121del (Y121del) and Tyr122del (Y122del).

2. A gene according to claim 1, which comprises the exons of the Nurr1 gene.

3. A gene according to claim 1 or 2, which comprises exon 3 of the Nurr1 gene.

4. A gene according to any one of claims 1-3, which comprises the mutation Met97Val.

5. A gene according to any one of claims 1-3, which comprises the mutation His103Arg.

6. A gene according to any one of claims 1-3, which comprises the mutation Tyr121del or Tyr122del.

7. A protein or a peptide encoded by a gene according to any one of claims 1-6.

8. A protein or peptide according to claim 7, which includes a Val residue in the position corresponding to amino acid no. 97 of the wild type Nurr1 protein.

9. A protein or peptide according to claim 7, which includes an Arg residue in the position corresponding to amino acid no. 103 of the wild type Nurr1 protein.

10. A protein or peptide according to claim 7, which does not include any Tyr residue in the position corresponding to amino acid no. 121 or 122 of the wild type Nurr1 protein.

11. An antibody raised against a protein or peptide according to any one of claims 7- 10, wherein said antibody specifically binds to said protein or peptide.

12. A transgenic, non-human animal, such as a mouse or a rat, comprising a gene according to any one of claims 1-6.

13. An antibody according to claim 11 for use as a medicament.

14. Use of an antibody according to claim 13 in the manufacture of a medicament for the treatment and/or prevention of psychotic conditions, such as schizophrenia and/or manic depressive disorder.

15. A pharmaceutical preparation comprising an antibody according to claim 11 together with a pharmaceutically acceptable carrier.

## Patentansprüche

1. Isoliertes Nurr1-Gen, enthaltend eine oder mehrere Mutationen, ausgewählt aus der Gruppe bestehend aus Met97Val (M97V), His103Arg (H103R), Tyr121del (Y121del) und Tyr122del (Y122del).

2. Gen nach Anspruch 1, das die Exons des Nurr1-Gens umfaßt.

3. Gen nach Anspruch 1 oder 2, das Exon 3 des Nurr1-Gens umfaßt.

4. Gen nach einem der Ansprüche 1-3, das die Mutation Met97Val umfaßt.

5. Gen nach einem der Ansprüche 1-3, das die Mutation His103Arg umfaßt.

6. Gen nach einem der Ansprüche 1-3, das die Mutation Tyr121del oder Tyr122del umfaßt.

7. Protein oder Peptid, codiert von einem Gen nach einem der Ansprüche 1-6.

8. Protein oder Peptid nach Anspruch 7, das an der Aminosäure Nr. 97 des Nurr1-Wildtypproteins entsprechenden Position einen Val-Rest enthält.

9. Protein oder Peptid nach Anspruch 7, das an der Aminosäure Nr. 103 des Nurr1-Wildtypproteins entsprechenden Position einen Arg-Rest enthält.

10. Protein oder Peptid nach Anspruch 7, das an der Aminosäure Nr. 121 oder 122 des Nurr1-Wildtypproteins entsprechenden Position keinen Tyr-Rest enthält.

11. Antikörper, erzeugt gegen ein Protein oder Peptid nach einem der Ansprüche 7-10, wobei der Antikörper spezifisch an das Protein oder Peptid bindet.

12. Transgenes, nicht menschliches Tier, wie beispielsweise eine Maus oder eine Ratte, umfassend ein Gen nach einem der Ansprüche 1-6.

13. Antikörper nach Anspruch 11 zur Verwendung als Arzneimittel.

14. Verwendung eines Antikörpers nach Anspruch 13 bei der Herstellung eines Arzneimittels zur Behandlung und/oder Vorbeugung von psychotischen Leiden, wie beispielsweise Schizophrenie und/oder manischdepressiver Erkrankung.

15. Pharmazeutische Zubereitung, umfassend einen Antikörper nach Anspruch 11 zusammen mit einem pharmazeutisch annehmbaren Träger.

## Revendications

1. Gène Nurr1 isolé comprenant une ou plusieurs mutations choisies dans le groupe constitué de Met97Val (M97V), His103Arg (H103R), Tyr121del (Y121del) et Tyr122del (Y122del).

2. Gène selon la revendication 1, qui comprend les exons du gène Nurr1.

3. Gène selon la revendication 1 ou 2, qui comprend l'exon 3 du gène Nurr1.

4. Gène selon l'une quelconque des revendications 1 à 3, qui comprend la mutation Met97Val.

5. Gène selon l'une quelconque des revendications 1 à 3, qui comprend la mutation His103Arg.

6. Gène selon l'une quelconque des revendications 1 à 3, qui comprend la mutation Tyr121del ou Tyr122del.

7. Protéine ou peptide codé par un gène selon l'une quelconque des revendications 1 à 6.

8. Protéine ou peptide selon la revendication 7, qui comprend un résidu Val en la position correspondant à l'acide aminé n° 97 de la protéine Nurr1 sauvage.

9. Protéine ou peptide selon la revendication 7, qui comprend un résidu Arg en la position correspondant à l'acide aminé n° 103 de la protéine Nurr1 sauvage.

10. Protéine ou peptide selon la revendication 7, qui ne comprend aucun résidu Tyr en la position correspondant à l'acide aminé, n° 121 ou 122 de la protéine Nurr1 sauvage.

11. Anticorps dirigé contre une protéine ou un peptide selon l'une quelconque des revendications 7 à 10, **caractérisé en ce que** ledit anticorps se lie de manière spécifique à ladite protéine ou audit peptide.

12. Animal transgénique non humain, tel qu'une souris ou un rat, comprenant un gène selon l'une quelconque des revendications 1 à 6.

13. Anticorps selon la revendication 11, destiné à une utilisation en tant que médicament.

14. Utilisation d'un anticorps selon la revendication 13, dans la fabrication d'un médicament destiné au traitement et/ou à la prévention de troubles psychotiques, tels que la schizophrénie et/ou la psychose maniaco-dépressive.

15. Préparation pharmaceutique comprenant un anticorps selon la revendication 11, conjointement avec un support pharmaceutiquement acceptable.
